# EUROPÄISCHE PATENTSCHRIFT

(11) **EP 2 352 532 B1**
(45) Veröffentlichungstag und Bekanntmachung des Hinweises auf die Patenterteilung: **18.10.2017**
(21) Anmeldenummer: 09740039.4
(22) Anmeldetag: 13.09.2009
(51) Int. Cl.: A61M 1/02, A61M 1/36, B04B 5/04

(54) **VERFAHREN UND VORRICHTUNG ZUR TRENNUNG VON BLUT UNTER EINSATZ EINER ZENTRIFUGE**
METHOD AND DEVICE FOR SEPARATING BLOOD USING A CENTRIFUGE
PROCÉDÉ ET DISPOSITIF DE SÉPARATION DE SANG AU MOYEN D'UNE CENTRIFUGEUSE

(30) Priorität: 12.09.2008 DE 102008047068
(43) Veröffentlichungstag der Anmeldung: 10.08.2011
(73) Patentinhaber: Pobitschka, Walter, 61350 Bad Homburg (DE)
(72) Erfinder: Pobitschka, Walter, 61350 Bad Homburg (DE)
(74) Vertreter: Jendricke, Susann
(86) Internationale Anmeldenummer: PCT/DE2009/001273
(87) Internationale Veröffentlichungsnummer: WO 2010/028638

(56) Entgegenhaltungen:
- EP-A1- 0 233 112
- WO-A1-00/07642
- WO-A1-00/38762
- WO-A1-84/02091
- WO-A1-99/45851
- DE-A1- 2 741 398
- DE-U1-202007 007 136
- US-A- 6 068 970

## Beschreibung

Die Erfindung betrifft ein Verfahren zur Trennung von Blut unter Einsatz einer Zentrifuge, wobei verschiedene Blutfraktionen gewonnen werden, wobei die Befüllung eines in die Zentrifuge einzubringenden Abtrennbehälters mit Blut über dessen steril konnektierbaren Zuführanschluss erfolgt, wobei die Entnahme der interessierenden Blutfraktion aus dem Abtrennbehälter über dessen steril konnektierbaren Entnahmeanschluss erfolgt und wobei eine Zuführeinrichtung und eine Entnahmeeinrichtung während der sterilen Verbindung mit dem Abtrennbehälter jeweils ein geschlossenes System ausbilden.

Des weiteren betrifft die Erfindung eine Vorrichtung zur Trennung von Blut unter Einsatz einer Zentrifuge mit einem Rotor, insbesondere zur Durchführung des Verfahrens, mit mindestens einem Abtrennbehälter, wobei der Abtrennbehälter mindestens einen steril konnektierbaren Zuführanschluss und mindestens einen steril konnektierbaren Entnahmeanschluss aufweist, an die gegen die Atmosphäre abgedichtete Zuführ- und Entnahmeeinrichtungen steril angeschlossen sind, wobei die Zuführeinrichtung und die Entnahmeeinrichtung geschlossene Systeme sind und während der sterilen Konnektierung mit den entsprechenden Anschlüssen des Abtrennbehälters mit diesem gemeinsam ebenfalls ein geschlossenes System ausbilden.

Blut kann mittels einer Zentrifuge in verschiedene Blutfraktionen getrennt werden, indem die Zentrifuge bei bestimmten Umdrehungen und über eine bestimmte Zeitdauer schnell gedreht wird. Nach dem Zentrifugieren hat sich das Blut im Abtrennbehälter verändert. Die einzelnen Bestandteile haben sich aufgeteilt in Blutplasma, das vor allem aus Eiweiß und Wasser besteht, weiße Blutkörperchen und feste Blutbestandteile.

Die Präparation des Blutes vor und nach dem Abtrennvorgang ist sehr aufwendig und erfolgt in der Regel in der Atmosphäre, also in einem offenen System, das zwangsläufig kontaminiert ist. Gerade bei Nabelschnurpräparaten, aber auch bei sonstigem Blut, ist die Kontamination unerwünscht. Für weitergehende Präparationen sind die weißen Blutkörperchen am interessantesten. WO 84/02091 A1 offenbart ein Verfahren und eine Vorrichtung zur Trennung von Blut unter Einsatz einer Zentrifuge, wobei verschiedene Blutfraktionen (Plasma, Buffy-Coat, Erythrozyten) gewonnen werden.

Ausgehend von dem aus der Praxis bekannten Stand der Technik liegt der Erfindung die Aufgabe zugrunde, ein Verfahren und eine Vorrichtung der in Rede stehenden Art anzugeben, wobei das Kontaminationsrisiko beim Befüllen mit Blut und bei der Entnahme von Blutfraktionen ausgeschlossen wird.
Die voranstehende Aufgabe wird im Hinblick auf das Verfahren durch die Merkmale des Patentanspruches 1 gelöst. Danach ist ein Verfahren der in Rede stehenden Art derart ausgestaltet, dass sich die Blutfraktion der weißen Blutkörperchen während des Zentrifugierens im wesentlichen innerhalb einer Strömungsverbindung zwischen zwei Behältnissen innerhalb des Abtrennbehälters sammelt und von dort mittels einer im Inneren der Entnahmeeinrichtung angeordneten Innenleitung entnommen wird.
Die voranstehende Aufgabe wird im Hinblick auf die Vorrichtung durch die Merkmale des Patentanspruches 15 gelöst. Danach ist eine Vorrichtung der in Rede stehenden Art derart ausgestaltet, dass der Abtrennbehälter zwei Behältnisse umfasst, die miteinander über eine Strömungsverbindung strömungsverbunden sind und dass die Entnahmeeinrichtung eine Innenleitung aufweist, die in die Strömungsverbindung und in das Behältnis applizierbar ist.
Ausgehend von dem aus der Praxis bekannten Stand der Technik ist erkannt worden, dass das Kontaminationsrisiko sehr stark vermindert werden kann, wenn die Befüllung des bekanntermaßen nach außen dichten Abtrennbehälters der Zentrifuge und die Entnahme daraus unter Ausschluss der Atmosphäre stattfindet. Erfindungsgemäß ist erkannt worden, dass der Kontakt mit der Atmosphäre ausgeschlossen werden kann, wenn zwischen der Zuführeinrichtung und dem Abtrennbehälter und der Entnahmeeinrichtung und dem Abtrennbehälter ein geschlossenes System ausgebildet wird, bzw. wenn über steril konnektierbare Anschlüsse die Voraussetzung dafür geschaffen wird, dass speziell ausgestaltete, gegen die Atmosphäre abgedichtete Zuführ- und Entnahmeeinrichtungen steril angeschlossen werden können, wobei im Inneren der Entnahmeeinrichtung eine Innenleitung angeordnet ist. Diese Innenleitung wird erfindungsgemäß nach der Verbindung der beiden geschlossenen Systeme zu einem gesamten geschlossenen System in den Abtrennbehälter, bzw. in die Strömungsverbindung eingeführt, um die Blutfraktion der weißen Blutkörperchen zu entnehmen. Es ist offensichtlich, dass in erfindungsgemäßer Weise das Kontaminationsrisiko verringert wird.

Nach einem bevorzugten Ausführungsbeispiel könnten zur Zuführung zwei Zuführeinrichtungen an zwei Zuführansschlüsse angeschlossen werden. Hierdurch wird die Möglichkeit eröffnet, zusätzlich zum Blut auch Hilfsmittel zur Verbesserung der Zentrifugierung zu verabreichen. Es könnte also eine Zuführeinrichtung für antikoaguliertes Blut und eine Zuführeinrichtung für Hydroxyethylstärkelösung, im weiteren mit HES bezeichnet, steril an jeweils einen Zuführanschluss angeschweißt werden. HES fördert die Sedimentation der Erythrozyten bzw. der roten Blutkörperchen, die sich absetzen sollen.

Während der Abtrennbehälter mit Blut befüllt wird, könnte in vorteilhafter Weise gleichfalls die Zuführeinrichtung für HES angeschlossen sein. Dies hat den Vorteil, dass die durch das Blut aus dem Abtrennbehälter verdrängte sterile Luft in die Zuführeinrichtung für HES gelangen kann. Darüber hinaus könnte während der Blutzuführung mittels der Zuführeinrichtung für HES auch ein Unterdruck erzeugt werden und die sterile Luft aktiv aus dem Abtrennbehälter angesaugt werden. Die verdrängte oder angesaugte sterile Luft könnte in einen Vorratsbehälter der Zuführeinrichtung für HES gelangen oder in eine Druckausgleicheinrichtung entlassen werden. Dabei ist sicherzustellen, dass die sterile Luft so in den Vorratsbehälter der Zuführeinrichtung für HES gelangt, dass sie an HES vorbei strömt. Dazu könnte der HES-haltige Bereich des flexiblen Vorratsbehälters der Zuführeinrichtung für HES während der Zufuhr der sterilen Luft von der Eintrittstelle zum Vorratsbehälter in Abstand gebracht werden. Bspw. könnte der als flexibler Beutel vorliegende Vorratsbehälter weggeknickt werden, so dass die verdrängte sterile Luft einströmt, ohne HES zu durchströmen. Für den Fall der HES-Zugabe könnte durch HES verdrängte sterile Luft genausgut in die Zuführrichtung für Blut gelangen.

Durch die besondere Ausgestaltung der Zuführeinrichtung könnte das Blut volumengenau zugegeben werden. Dasselbe gilt für die Zugabe von HES. Die erforderliche Menge an HES könnte unter Berücksichtigung der zugeführten Blutmenge zuvor berechnet werden.
Wenn die Befüllung mit Blut ggf. zusätzlich mit HES, abgeschlossen ist, könnte eine sterile Diskonnektierung zwischen Zuführeinrichtung und Abtrennbehälter erfolgen. Dies könnte durch das für sich bekannte Steril Welding-Verfahren realisiert werden. Wesentlich ist jedenfalls, dass der Abtrennbehälter von der Außenwelt abgeschottet bleibt.
Der Abtrennbehälter hat beim Zentrifugieren viele Kräfte aufzunehmen und ist deshalb zur Stabilisierung in einem Mantelgerüst angeordnet. Das Mantelgerüst könnte eine Deckplatte umfassen, die Ausnehmungen für die Aufnahme der Zuführanschlüsse und ggf. des Entnahmeanschlusses aufweist. Nach der Diskonnektierung, können die frei abragenden Anschlüsse in die Ausnehmungen eingebracht werden, damit beim Zentrifugieren keine Störungen auftreten. Es wird zeitgenau, insbesondere bei einer Beschleunigung von 1500 g, zentrifugiert.

Innerhalb des Abtrennbehälters sind zwei miteinander strömungsverbundene Behältnisse vorgesehen. Während des Zentrifugierens sammelt sich die Blut-fraktion der weißen Blutkörperchen hauptsächlich innerhalb der Strömungsverbindung zwischen den Behältnissen. Dies ist an der Farbe der Substanz erkennbar. Zusätzlich könnte ein optisches Lesegerät zu Hilfe genommen werden, um die Phasengrenze zu ermitteln. Wenn die Phasengrenze erkannt ist, könnte die als Kunststoffschlauch vorliegende Strömungsverbindung mittels Schlauchklemme an der Phasengrenze zwischen der Blutfraktion der festen Blutbestanteile zu der Blutfraktion der weißen Blutkörperchen abgeklemmt werden. Die Schlauchklemme könnte über die offenen Bereiche des Mantelgerüsts appliziert werden. Der Abtrennbehälter selbst ist luftdicht verschlossen und nur über die Anschlüsse mit ebenfalls geschlossenen Systemen konnektierbar. Die Ausbildung der Strömungsverbindung per Kunststoffschlauch ist wegen des leichten Abklemmens von Vorteil. Dabei kann die Schlauchklemme genau dort positioniert werden, wo die Phasengrenze zwischen Erythrozyten und Leukozyten verläuft. Dies kann bei jedem Patienten anders sein.

Erfahrungen haben gezeigt, dass bei dieser Formgebung des Abtrennbehälters mit dem Kunststoffschlauch die Ausbeute sehr hoch ist. Außerdem muss sich die Entnahme von Blutfraktionen nicht auf die Leukozyten beschränken. Vielmehr kann zum Erreichen eines standardisierten Volumens auch Plasma aus dem oberen inneren Behältnis abgesaugt werden. Das standardisierte Volumen findet bei der Kryokonservierung Anwendung, wobei enge Vorgaben bei der Durchführung existieren.

Nach einem zweiten Ausführungsbeispiel könnte noch ein weiterer Zuführanschluss für eine weitere Zuführeinrichtung vorgesehen sein, hier zur Zuführung eines Füllstoffes zur Verkleinerung des Volumens des Behälters, der die zentrifugierten Erythrozyten aufnimmt. Damit die Phasengrenze innerhalb der Strömungsverbindung, vorzugsweise am unteren Ende der Strömungsverbindung, zwischen den Behältnissen ausgebildet wird, könnte das Volumen des unteren Behälters verändert werden. Die Veränderung hängt damit zusammen, dass ein Unterschied zwischen dem Füllvolumen des Blutes und der darin enthaltenen Erythrozyten und dem Packvolumen der zentrifugierten Erythrozyten besteht. Anhand des für jede Blutprobe bekannten Hämatokritwertes und anhand des Füllvolumens des Blutes, kann das künftige Packvolumen berechnet werden. Das Füllvolumen kann entweder dadurch ermittelt werden, dass der Wert des bekannten Volumens der Zuführeinrichtung (bspw. Beutelvolumen) verwendet wird oder dass eine skalierte Zuführeinrichtung verwendet wird und dadurch die Menge an entnommen Blut vorliegt, die dann das Füllvolumen ergibt. Alternativ kann auch der Abtrennbehälter skaliert sein. Die Verkleinerung des Volumens des Behälters des Abtrennbehälters, das für die Aufnahme der zentrifugierten Erythrozyten vorgesehen ist, könnte durch die Befüllung mit inerten Kügelchen herbeigeführt werden. Nach Bestimmung des Füllvolumens im Abtrennbehälter und nach Berechnung des Packvolumens unter Zuhilfenahme des Hämatokritwertes kann abgeschätzt werden, zu wieviel Prozent der untere Behälter an zentrifugierten Erythrozyten ausgefüllt werden wird. Das zu erwartende Packvolumen der Erythrozyten unterschreitet in der Regel das Volumen des unteren Behältnisses des Abtrennbehälters. Damit nun die Phasengrenze in der Strömungsverbindung ausgebildet wird und nicht im unteren Behältnis, könnten die Kügelchen in dem Maße eingefüllt werden, dass das über das erwartete Packvolumen hinausgehende Volumen aufgefüllt wird. Beim Zentrifugieren sind die Kügelchen aufgrund ihres vergleichsweise hohen Gewichts am Boden des in Rede stehenden Behälters gesammelt und die Erythrozyten darüber. Die Phasengrenze stellt sich, wie gewünscht, in der Strömungsverbindung, an deren unteren Ende ein. So können sich Leukozyten, bzw. das sogenannte Buffycoat, bestehend aus Leukozyten und Thrombozyten, optimal in der Strömungsverbindung ansammeln.
Zur Entnahme der Blutfraktion der weißen Blutkörperchen aus dem Kunststoffschlauch wird erfindungsgemäß eine Entnahmeeinrichtung mit dem Entnahmeanschluss steril konnektiert. Die Entnahmeeinrichtung könnte eine in DE 10 2008 035 835 beschriebene Ausgestaltung aufweisen. Innerhalb der Entnahmeeinrichtung könnte eine Spritze angeordnet sein, über deren Kolbenhub die interessierende Blutfraktion angesaugt und in die gegen die Atmosphäre abgedichtete Entnahmeeinrichtung transferiert werden. Zur Aufnahme der Blutfraktion könnte innerhalb der Entnahmeeinrichtung bspw. ein Behälter vorgesehen sein.
Auch die Entnahmeinrichtung könnte nach erfolgter Entnahme der Blutfraktion der weißen Blutkörperchen aus dem Abtrennbehälter steril diskonnektiert werden, so dass sichergestellt ist, dass die Entnahmeeinrichtung keinesfalls mit der Atmosphäre in Kontakt kommt.
Im Hinblick auf die Ausgestaltung der Vorrichtung ist zunächst die besondere Formgebung des Abtrennbehälters gemäß einem bevorzugten Ausführungsbeispiel hervorzuheben. Im Inneren des Abtrennbehälters fasst der Abtrennbehälter zwei Be-hältnisse um, die miteinander strömungsverbunden sind. Wenn sich der Ab-trennbehälter in der Rotorschüssel des Rotors in Ruhestellung befindet, könnte das erste Behältnis mit einem trichterförmigen Boden oberhalb des zweiten Behältnisses angeordnet sein. Am Trichterauslass könnte die Strömungsverbindung in Form eines Kunststoffschlauches angeschlossen sein, die in das zweite Behältnis mündet. Der trichterförmige Boden erleichtert die Zuführung von Blut vom ersten zum zweiten Behältnis. Alternativ zu der hier beschriebenen Formgebung könnte auch ein ringförmiger Abtrennbehälter vorgesehen sein, der sich um die Rotorachse erstreckt und einen Querschnitt aufweist, der sich aus zwei Erweiterungen und einer mittigen Verengung zusammensetzt. Weiterführend könnte der ringförmige Abtrennbehälter auch in eine Vielzahl von Kammern segmentiert sein.

Da die Blutfraktion der weißen Blutkörperchen von Interesse für nachfolgende Präparationsschritte ist und diese nach der Zentrifugierung hauptsächlich in der Strömungsverbindung gesammelt sind, könnte der Entnahmeanschluss zur Strömungsverbindung fluchten. Auf diese Weise wird die Einführung einer Innenleitung der Entnahmeeinrichtung nach steriler Konnektierung am Entnahmeanschluss erheblich vereinfacht.

Aufgrund des weichen Kunststoffschlauches als Strömungsverbindung ist die Stabilität des Abtrennbehälters nicht besonders hoch. Deshalb könnte der Abtrennbehälter innerhalb eines Mantelgerüsts angeordnet sein. Das Mantelgerüst könnte offene Bereiche aufweisen, durch die hindurch, an der Strömungsverbindung bzw. am Kunststoffschlauch eine Schlauchklemme befestigbar ist. Die Schlauchklemme soll verhindern, dass eine Vermischung der nach der Zentrifugierung gewonnenen Phasen - rote Blutkörperchen im unteren Innenbehältnis und weiße Blutkörperchen im Kunststoffschlauch bzw. in der Strömungsverbindung stattfindet.

Wie bereits im Hinblick auf das Verfahren ausgeführt, könnten nach einem ersten Ausführungsbeispiel zwei zum Entnahmeanschluss beabstandete Zuführanschlüsse für Blut einerseits und für Hydroxyethylstärkelösung HES andererseits vorgesehen sein. Konkret könnte eine Zuführeinrichtung verwendet werden, die eine innerhalb einer Umhüllung sitzende Spritze mit einem Tubus und mit einem Kolben umfasst, der eine Durchtrittsöffnung aufweist. Wenn zusätzlich noch Absperreinrichtungen am Tubus und am Kolbenende vorgesehen sind, kann mehrmals angesaugt und ausgestoßen werden, wobei die Absperreinrichtungen entweder jeweils abwechselnd geschlossen oder geöffnet sind. Hier kann auch in beiden Richtungen gearbeitet werden, indem das Ansaugen nicht zwangsläufig durch Ausfahren des Kolbens aus dem Tubus erfolgen muss. Durch die Durchtrittsöffnung im Kolben kann der Saugvorgang auch durch Einfahren stattfinden, es muss nur die richtige Absperreinrichtung geöffnet werden, nämlich die auf der Saugseite. Weiter ist von Wichtigkeit, dass von der Umhüllung steril konnektierbare Anschlüsse abragen. Weitere Möglichkeiten für geschlossene Gebersysteme mit abragenden, steril konnektierbaren Anschlüssen ergeben sich aus DE 10 2008 035 837.

Nach einem zweiten Ausführungsbeispiel könnte der Abtrennbehälter einen weiteren steril konnektierbaren Zuführanschluss aufweisen, an den eine weitere gegen die Atmosphäre abgedichtete Zuführeinrichtung für einen Füllstoff, insbesondere inerte Kügelchen, zur Volumenverminderung in dem Behälter, der zur Aufnahme der zentrifugierten Erythrozyten vorgesehen ist. Die Zuführeinrichtungen könnten gravitationskraftbetrieben sein und jeweils eine Dosierabsperrung aufweisen, damit volumengenau dosiert werden kann.

Gemäß einem weiteren Ausführungsbeispiel für eine Entnahmeeinrichtung mit der schon beschriebenen, in die Strömungsverbindung applizierbaren Innenleitung könnte diese innerhalb einer Umhüllung mit einer Spritze mit einem Tubus und mit einem Kolben in Verbindung stehen, der eine Durchtrittsöffnung aufweist. Die Handhabung der Innenleitung kann über einen Faltenbalg der Umhüllung erfolgen, zudem bilden die steril konnektierten Anschlüsse die Führung für die Innenleitung aus. Die Handhabung der Spritze erfolgt dann über Absperreinrichtungen wie im Zusammenhang mit der Zuführeinrichtung beschrieben.

Für die Erfindung ist von wesentlicher Bedeutung, dass die Zuführeinrichtung und die Entnahmeeinrichtung für sich geschlossene Systeme sind, die während der sterilen Konnektierung mit dem entsprechenden Anschluss des Abtrennbehälters mit diesem gemeinsam ebenfalls ein geschlossenes System ausbilden. Hierdurch spielt die Kontamination keine Rolle mehr.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung zweier Ausführungsbeispiele der Erfindung anhand der Zeichnung zu verweisen. In Verbindung mit der Erläuterung des angeführten Ausführungsbeispiels der Erfindung werden auch im allgemeinen bevorzugte Ausgestaltungen und Weiterbildungen der Lehre erläutert. In der Zeichnung zeigen
- Fig. 1: als Prinzipskizze eine Draufsicht auf die Zentrifuge in Betriebsstellung unter Anwendung der erfindungsgemäßen Vorrichtung mit Abtrennbehältnis,
- Fig. 2: in schematischer Darstellung, vergrößert, ein Detail aus Fig. 1, betreffend ein erstes Ausführungsbeispiel der erfindungsgemäßen Vorrichtung mit zwei Zuführanschlüssen und einem Entnahmeanschluss,
- Fig. 3: in schematischer Darstellung, verkleinert, der Gegenstand aus Fig. 2 mit zwei steril konnektierten Zuführeinrichtungen,
- Fig. 4: eine Skizze zu einer alternativen Abführung von verdrängter steriler Luft aus dem Abtrennbehältnis,
- Fig. 5: in schematischer Darstellung, verkleinert, der Gegenstand aus Fig. 2 vor der sterilen Konnektierung mit einer Entnahmeeinrichtung,
- Fig. 6: in schematischer Darstellung, verkleinert, der Gegenstand aus Fig. 2 mit steril konnektierter Entnahmeeinrichtung und Schlauchklemme sowie eingeführter Innenleitung.
- Fig. 7: in schematischer Darstellung, Zuführeinrichtungen am Abtrennbehälter gemäß einem zweiten Ausführungsbeispiel der erfindungsgemäßen Vorrichtung.

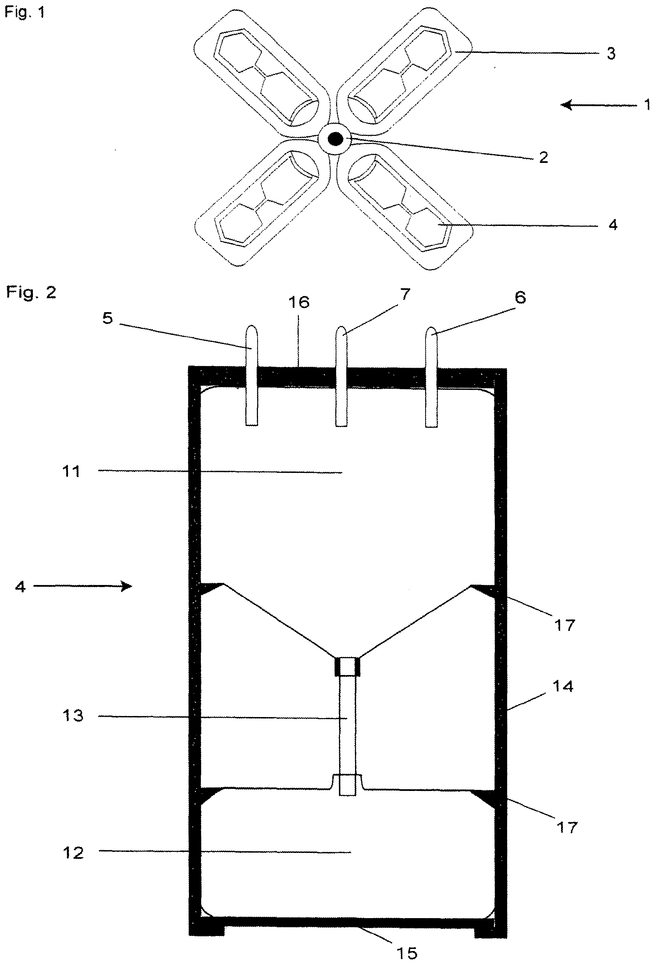

Die Figuren 1 bis 3, 5 und 6 zeigen eine Vorrichtung zur Trennung von Blut unter Einsatz einer in Fig. 1 dargestellten Zentrifuge 1 mit einem Rotor 2 und hier vier Rotorschüsseln 3. Für jede Rotorschüssel 3 ist ein Abtrennbehälter 4 vorgesehen. Auf die Darstellung von Rotorwelle und weiteren konstruktiven Merkmalen ist hier verzichtet.

Der Abtrennbehälter 4 weist gemäß dem ersten Ausführungsbeispiel zwei steril konnektierbare Zuführanschlüsse 5, 6 und einen steril konnektierbaren Entnahmeanschluss 7 auf, an die gegen die Atmosphäre abgedichtete Zuführ- und Entnahmeeinrichtungen 8, 9, 10 steril anschließbar sind.

Der Abtrennbehälter 4 umfasst zwei Behältnisse 11, 12, die miteinander strömungsverbunden sind. Der Boden des Behältnisses 11 ist als Trichter ausgebildet. Zur Ausbildung der Strömungsverbindung 13 dient ein flexibler PVC-Schlauch, der mit den Behältnissen 11, 12 dicht verschweißt ist. Der Entnahmeanschluss 7 fluchtet mit der Strömungsverbindung 13. Der Abtrennbehälter 4 ist innerhalb eines Mantelgerüsts 14 mit einer Grundplatte 15 und einer Deckplatte 16 angeordnet. Die Grundplatte 15 und die Deckplatte 16 sind mit dem Mantelgerüst fest verbunden. Das Mantelgerüst 14 verfügt über Verstärkungen 17, die die Funktion von Innenschultern oder Stabilisatoren erfüllen.

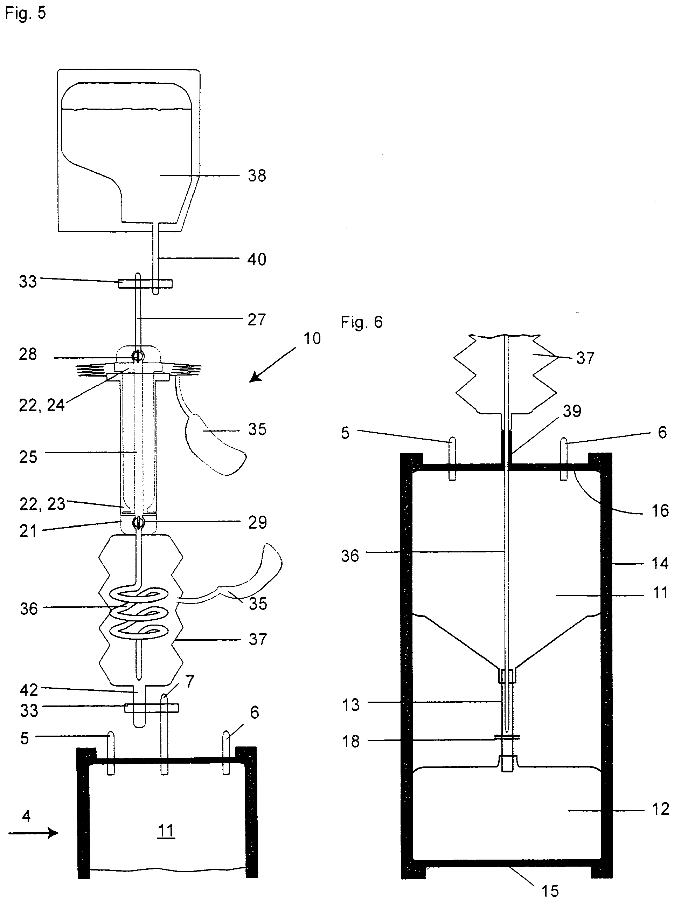

Das Mantelgerüst 14 verfügt über hier nicht dargestellte Öffnungen, durch die hindurch die in Fig. 6 gezeigte Schlauchklemme 18 an der Strömungsverbindung 13 festlegbar ist.

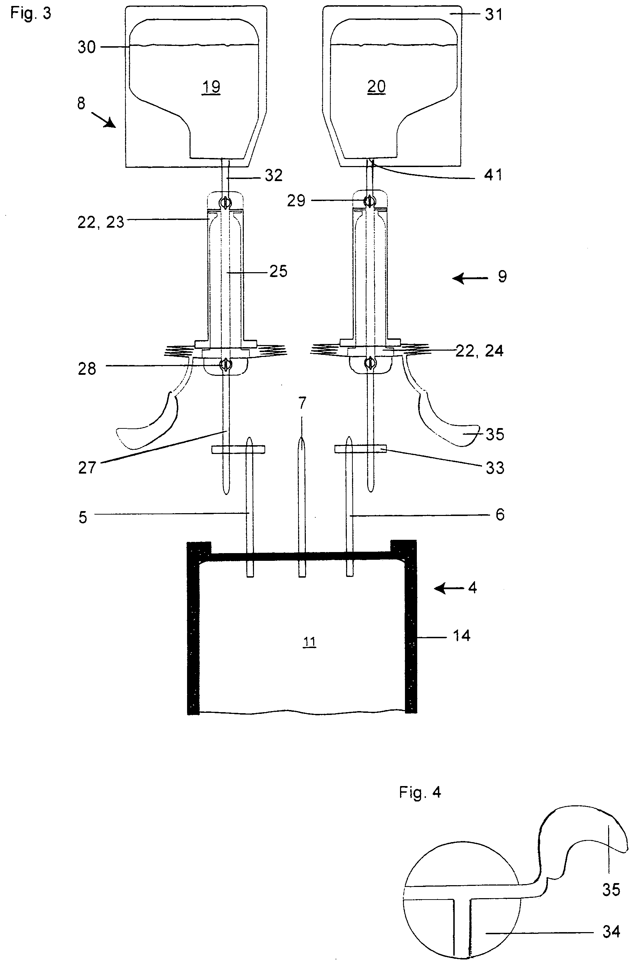

In Fig. 3 ist dargestellt, dass über die Zuführeinrichtung 8 Nabelschnurblut mit Antikoagulans 19 in den Abtrennbehälter 4 gelangt und über die Zuführeinrichtung 9 eine Hydroxyethylstärkelösung 20, im weiteren HES genannt.

Die Zuführeinrichtungen 8, 9 verfügen über eine innerhalb einer Umhüllung 21 sitzende Spritze 22 mit einem Tubus 23 und mit einem Kolben 24 umfasst, der eine Durchtrittsöffnung 25 aufweist. Mit 35 ist eine Druckausgleicheinrichtung bezeichnet, in die verdrängte sterile Luft entweichen kann.

Von der Umhüllung 21 ragen steril konnektierbare Anschlüsse 27 ab, die über Absperreinrichtungen 28, 29 in Form von Zweiwegehähnen öffen- und verschließbar sind. Die Absperreinrichtungen 28, 29 dienen zur Realisierung des Transfers des Nabelschnurblutes mit Antikoagulans 19 aus dem Vorratsbehälter 30 zum Abtrennbehälter 4 und zur Realisierung des Transfers des HES 20 aus dem Vorratsbehälter 31 zum Abtrennbehälter 4.

Über die Spritze 22 wird die jeweilige Substanz 19, 20 in der jeweiligen Zuführeinrichtung 8, 9 angesaugt. Durch die Durchtrittsöffnung 25 ist die jeweilige Substanz 19, 20 in den Abtrennbehälter 4 transferierbar. Der tubusseitige Anschluss 32 und der kolbenseitige Anschluss 27 ragen von der Umhüllung 21 ab und sind mit dieser dicht verbunden.

Zur Funktionsweise der Zuführeinrichtungen 8, 9 wird folgendes ausgeführt:

Im vorliegenden Ausführungsbeispiel sind die Vorratsbehälter 30, 31 und die Spritze 22 mit der Umhüllung 21 vorkonfektioniert. Der kolbenseitige Anschluss 27 wird an den Zuführanschluss 5, 6 des Abtrennbehälters 4 angeschlossen.

Die Absperreinrichtung 29 des tubusseitigen Anschlusses 32 wird vor dem, hier volumengenauen, Ansaugen geöffnet und die Absperreinrichtung 28 des kolbenseitigen Anschlusses 27 der Spritze 22 wird geschlossen. Die Spritze 22 bzw. der Kolben 24 und die Absperreinrichtungen 28, 29 werden von außen über die Umhüllung 21 betätigt.

Beim Ansaugen wird der Tubus 23 der Spritze 22 mit der Substanz 19, 20 befüllt.

Vor dem Weiterleiten der Substanz 19, 20 aus der Spritze 22 zum Abtrennbehälter 4 wird die Absperreinrichtung 29 des tubusseitigen Anschlusses 32 geschlossen und die Absperreinrichtung 28 des kolbenseitigen Anschlusses 27 geöffnet. Zum Weiterleiten der Substanz 19, 20 wird der Kolben 24 der Spritze 22 in den Tubus 23 eingefahren, wobei die Entleerung des Tubus 23 über die Durchtrittsöffnung 25 und den kolbenseitigen Anschluss 27 und die dortige geöffnete Absperreinrichtung 28 in den Zuführanschluss 5, 6 und in den Abtrennbehälter 4 erfolgt. Mit 33 ist die sterile Verbindung zwischen den Zuführanschlüssen 5, 6 sowie den kolbenseitigen Anschlüssen 27 bezeichnet.

Fig. 4 zeigt als Alternative zur Absperreinrichtung 29, 28 in Form eines Zweiwegehahns einen Dreiwegehahn 34 mit einer Druckausgleicheinrichtung 35 zur Aufnahme von verdrängter steriler Luft aus dem Abtrennbehälter 4. Auch die Zuführeinrichtungen 8, 9 und die Entnahmeeinrichtung10 verfügen über eine Druckausgleicheinrichtung 35, die Volumenänderungen abfängt.

Die Fig. 5 und 6 befassen sich mit dem Entnahmevorgang nach der Zentrifugation. Die Zuführeinrichtungen 8, 9 sind bereits steril abgeschweißt, die Zuführanschlüsse 5, 6 sind ein wenig kürzer geworden. Die Entnahmeeinrichtung 10 weist eine Innenleitung 36 auf, die durch den Entnahmeanschluss 7 in die Strömungsverbindung 13 applizierbar ist. Die Innenleitung 36 ist innerhalb eines Faltenbalges 37 mit Druckausgleicheinrichtung 35 angeordnet und schließt steril an die Umhüllung 21 mit einer Spritze 22 mit einem Tubus 23 und mit einem Kolben 24 an, der eine Durchtrittsöffnung 25 aufweist. Die Spritze 22 der Entnahmeeinrichtung 10 funktioniert nach dem selben Prinzip wie die Spritze der Zuführeinrichtungen 8, 9. Auch hier sind Absperreinrichtungen 28, 29 vorgesehen. Unterschied ist nur, dass der Tubus 23 zum Abtrennbehälter 4 weist, da dort der Ansaugvorgang nebst entsprechender Betätigung der Absperreinrichtungen 28, 29 stattfindet, bevor die Entleerung der entnommenen Blutfraktion der weißen Blutkörperchen über die Durchtrittsöffnung 25 des Kolbens 24 in den kollabierten Beutel 38 erfolgt. Der Beutel 38 ist ebenfalls über seinen Anschluss 40 steril mit dem kolbenseitigen Anschluss 27 verbunden.

In Fig. 5 ist die Innenleitung 36 noch aufgewickelt. In Fig. 6 ist die Innenleitung 36 ausgefahren und in die Strömungsverbindung 13 eingeführt, in der sich die hier interessierenden weißen Blutkörperchen gesammelt haben. Fig. 6 zeigt außerdem noch eine Stabilisierungshülse 39 am Entnahmeanschluss 7.

Die Zuführeinrichtungen 8, 9 und die Entnahmeeinrichtung 10 sind geschlossene Systeme wie auch der Abtrennbehälter 4. Während der sterilen Konnektierung mit den entsprechenden Anschlüssen 5, 6, 7 des Abtrennbehälters 4 bilden die Zuführeinrichtungen 8, 9 und die Entnahmeeinrichtung 10 mit diesem gemeinsam ebenfalls ein geschlossenes System aus.

Mittels der Vorrichtung können folgende Verfahrensschritte ausgeführt werden:

Es werden über die beiden Zuführeinrichtungen 8, 9 Nabelschnurblut mit Antikoagulans 19 und HES 20 in den Abtrennbehälter 4 eingeführt. Dabei gelangt sterile Luft aus dem Abtrennbehälter 4 in die Zuführeinrichtung 9 für HES 20 und zwar bis in den Vorratsbehälter 31. Bei Fig. 4 wird alternativ ein Dreiwegehahn 34 mit einer Druckausgleicheinrichtung 35 zur Verfügung gestellt. Die verdrängte sterile Luft gelangt so in den Vorratsbehälter 31 der Zuführeinrichtung 9 für HES 20, dass sie an HES 20 vorbei strömt. Der HES-haltige Bereich des flexiblen Vorratsbehälters 31 der Zuführeinrichtung 9 für HES 20 wird während der Zufuhr der sterilen Luft von der Eintrittstelle 42 in den Vorratsbehälter 31 in Abstand gebracht. Es erfolgt eine volumengenau Zugabe der beiden Substanzen 19, 20. Die erforderliche Menge an HES 20 wird unter Berücksichtigung der zugeführten Menge an Nabelschnurblut mit Antikoagulans 19 berechnet. Die Zuführeinrichtungen 8, 9 werden nach erfolgter Befüllung des Abtrennbehälters 4 steril diskonnektiert. Zur Entnahme der Blutfraktion der weißen Blutkörperchen aus der Strömungsverbindung 13 wird die Entnahmeeinrichtung 10 mit dem Entnahmeanschluss 7 steril konnektiert. Die im Inneren der Entnahmeeinrichtung 10 angeordnete Innenleitung 36 wird zur Entnahme der Blutfraktion der weißen Blutkörperchen in die Strömungsverbindung 13 eingebracht. Nachdem die Blutfraktion der weißen Blutkörperchen durch wiederholtes Ansaugen und Ausstoßen aus dem Abtrennbehälter entnommen wurde, werden der Entnahmeanschluss 7 und der Anschluss 42 des Faltenbalges 37 der Entnahmeeinrichtung 10 steril diskonnektiert.

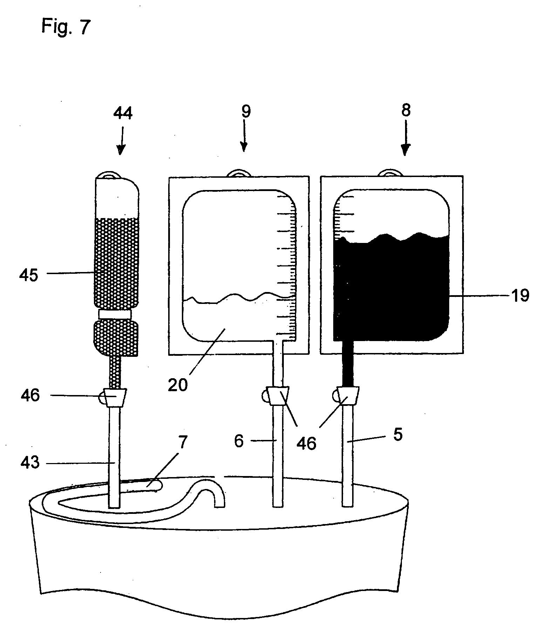

Das in Fig. 7 gezeigte zweite Ausführungsbeispiel der erfindungsgemäßen Vorrichtung bezieht sich auf einen dritten steril konnektierbaren Zuführanschluss 43, an den eine weitere gegen die Atmosphäre abgedichtete Zuführeinrichtung 44 zur Zuführung von inerten Kügelchen 45 angeschlossen ist. Die inerten Kügelchen 45 werden zur Volumenverminderung in Behälter 12 benötigt, damit die Phasengrenze zwischen den Erythrozyten, die sich nach der Zentrifugation vor allem in Behälter 12 sammeln sollen und den Leukozyten, die sich nach der Zentrifugation ausschließlich in der Strömungsverbindung 13 sammeln sollen, eine vorbestimmte Position am unteren Ende der Strömungsverbindung13 erlangt.

Alle drei Zuführeinrichtungen 8, 9, 44 sind hier gravitationskraftbetrieben. Zur Dosierung von Blut 19, HES 20 und inerten Kügelchen 45 ist jeweils eine Dosierabsperrung 46 vorgesehen.

Hinsichtlich weiterer, in den Figuren nicht gezeigter Merkmale wird auf den allgemeinen Teil der Beschreibung verwiesen.

Abschließend sei darauf hingewiesen, dass die erfindungsgemäße Lehre nicht auf das voranstehend erörterte Ausführungsbeispiel eingeschränkt ist.

**Bezugszeichenliste**

| | | | |
|---|---|---|---|
| 1 | Zentrifuge | 43 | Zuführanschluss |
| 2 | Rotor | 44 | Zuführeinrichtung |
| 3 | Rotorschüssel | 45 | Inerte Kügelchen |
| 4 | Abtrennbehälter | 46 | Dosierabsperrung |
| 5 | Zuführanschluss | | |
| 6 | Zuführanschluss | | |
| 7 | Entnahmeanschluss | | |
| 8 | Zuführeinrichtung | | |
| 9 | Zuführeinrichtung | | |
| 10 | Entnahmeeinrichtung | | |
| 11 | Behälter | | |
| 12 | Behälter | | |
| 13 | Strömungsverbindung, Kunststoffschlauch | | |
| 14 | Mantelgerüst | | |
| 15 | Grundplatte | | |
| 16 | Deckplatte | | |
| 17 | Verstärkungen | | |
| 18 | Schlauchklemme | | |
| 19 | Nabelschnurblut + Antikoagulans, Substanz | | |
| 20 | HES, Substanz | | |
| 21 | Umhüllung | | |
| 22 | Spritze | | |
| 23 | Tubus | | |
| 24 | Kolben | | |
| 25 | Durchtrittsöffnung | | |
| 27 | Anschluss | | |
| 28 | Absperreinrichtung | | |
| 29 | Absperreinrichtung | | |
| 30 | Vorratsbehälter | | |
| 31 | Vorratsbehälter | | |
| 32 | Anschluss | | |
| 33 | Sterile Verbindung | | |
| 34 | Dreiwegehahn | | |
| 35 | Druckausgleicheinrichtung | | |
| 36 | Innenleitung | | |
| 37 | Faltenbalg | | |
| 38 | Beutel | | |
| 39 | Stabilisierungshülse | | |
| 40 | Anschluss | | |
| 41 | Eintrittstelle | | |
| 42 | Anschluss | | |

## Patentansprüche

1. Verfahren zur Trennung von Blut unter Einsatz einer Zentrifuge, wobei verschiedene Blutfraktionen gewonnen werden, wobei die Befüllung eines in die Zentrifuge einzubringenden Abtrennbehälters mit Blut über dessen steril konnektierbaren Zuführanschluss erfolgt, wobei die Entnahme der interessierenden Blutfraktion aus dem Abtrennbehälter über dessen steril konnektierbaren Entnahmeanschluss erfolgt und wobei eine Zuführeinrichtung und eine Entnahmeeinrichtung während der sterilen Verbindung mit dem Abtrennbehälter jeweils ein geschlossenes System ausbilden,
**dadurch gekennzeichnet,**
**dass** sich die Blutfraktion der weißen Blutkörperchen während des Zentrifugierens im wesentlichen innerhalb einer Strömungsverbindung zwischen zwei Behältnissen innerhalb des Abtrennbehälters sammelt und von dort mittels einer im Inneren der Entnahmeeinrichtung angeordneten Innenleitung entnommen wird.

2. Verfahren nach Anspruch 1, **dadurch gekennzeichnet, dass** zur Zuführung zwei Zuführeinrichtungen an zwei Zuführanschlüsse angeschlossen werden.

3. Verfahren nach Anspruch 2, **dadurch gekennzeichnet, dass** eine Zuführeinrichtung für Blut und eine Zuführeinrichtung für Hydroxyethylstärkelösung (HES) steril an jeweils einen Zuführanschluss angeschweißt werden.

4. Verfahren nach Anspruch 3, **dadurch gekennzeichnet, dass** während der Befüllung des Abtrennbehälters mit Blut, sterile Luft aus dem Abtrennbehälter in die Zuführeinrichtung für HES gelangt oder dass während der Befüllung des Abtrennbehälters mit HES, sterile Luft aus dem Abtrennbehälter in die Zuführeinrichtung für Blut gelangt.

5. Verfahren nach Anspruch 4, **dadurch gekennzeichnet, dass** während der Blutzuführung mittels der Zuführeinrichtung für HES ein Unterdruck erzeugt wird oder dass während der HES-Zuführung mittels der Zuführeinrichtung für Blut ein Unterdruck erzeugt wird.

6. Verfahren nach Anspruch 4 oder 5, **dadurch gekennzeichnet, dass** die verdrängte sterile Luft in einen Vorratsbehälter der Zuführeinrichtung für HES oder Blut gelangt oder in eine Druckausgleicheinrichtung entlassen wird.

7. Verfahren nach Anspruch 6, **dadurch gekennzeichnet, dass** die sterile Luft so in den Vorratsbehälter der Zuführeinrichtung für HES oder Blut gelangt, dass sie an HES oder Blut vorbei strömt.

8. Verfahren nach Anspruch 7, **dadurch gekennzeichnet, dass** der HES- oder bluthaltige Bereich des flexiblen Vorratsbehälters der Zuführeinrichtung für HES oder Blut während der Zuführung der sterilen Luft von der Eintrittstelle in den Vorratsbehälter in Abstand gebracht wird.

9. Verfahren nach einem der Ansprüche 1 bis 8, **dadurch gekennzeichnet, dass** die Zuführeinrichtung nach erfolgter Befüllung des Abtrennbehälters steril diskonnektiert wird.

10. Verfahren nach einem der Ansprüche 1 bis 9, **dadurch gekennzeichnet, dass** die als Kunststoffschlauch vorliegende Strömungsverbindung mittels Schlauchklemme dort abgeklemmt wird, wo sich die Phasengrenze zwischen der Blutfraktion der roten Blutkörperchen zu der Blutfraktion der weißen Blutkörperchen befindet.

11. Verfahren nach einem der Ansprüche 1 bis 10, **dadurch gekennzeichnet, dass** zusätzlich zur Entnahme der Blutfraktion der weißen Blutkörperchen, auch Plasma entnommen wird und dass die Entnahmeeinrichtung mit dem Entnahmeanschluss steril konnektiert wird.

12. Verfahren nach einem der Ansprüche 1 bis 11, **dadurch gekennzeichnet, dass** die Innenleitung über die zur Entnahme der Blutfraktion der weißen Blutkörperchen steril verbundenen Anschlüsse der Entnahmeeinrichtung und des Abtrennbehälters in die Strömungsverbindung eingebracht wird.

13. Verfahren nach einem der Ansprüche 1 bis 12, **dadurch gekennzeichnet, dass** die Blutfraktion der weißen Blutkörperchen, ggf. zusätzliches Plasma, über eine innerhalb der Entnahmeeinrichtung angeordnete Spritze und deren Kolbenhub angesaugt und in die gegen die Atmosphäre abgedichtete Entnahmeeinrichtung, insbesondere in einen innerhalb der Entnahmeeinrichtung angeordneten Behälter, transferiert wird.

14. Verfahren nach einem der Ansprüche 1 bis 13, **dadurch gekennzeichnet, dass** die Entnahmeinrichtung nach erfolgter Entnahme der Blutfraktion der weißen Blutkörperchen, ggf. Plasma, aus dem Abtrennbehälter vom Entnahmeanschluss steril diskonnektiert wird.

15. Vorrichtung zur Trennung von Blut unter Einsatz einer Zentrifuge (1) mit einem Rotor (2), insbesondere zur Durchführung des Verfahrens nach einem der Ansprüche 1 bis 14, mit mindestens einem Abtrennbehälter (4), wobei der Abtrennbehälter (4) mindestens einen steril konnektierbaren Zuführanschluss (5, 6) und mindestens einen steril konnektierbaren Entnahmeanschluss (7) aufweist, an die gegen die Atmosphäre abgedichtete Zuführ- und Entnahmeeinrichtungen (8, 9, 10, 44) steril angeschlossen sind, wobei die Zuführeinrichtung (8, 9, 44) und die Entnahmeeinrichtung (10) geschlossene Systeme sind und während der sterilen Konnektierung mit den entsprechenden Anschlüssen (5, 6, 43, 7) des Abtrennbehälters mit diesem gemeinsam ebenfalls ein geschlossenes System ausbilden,
**dadurch gekennzeichnet,**
**dass** der Abtrennbehälter (4) zwei Behältnisse (11, 12) umfasst, die miteinander über eine Strömungsverbindung (13) strömungsverbunden sind und dass die Entnahmeeinrichtung (10) eine Innenleitung (36) aufweist, die in die Strömungsverbindung (13) und in das Behältnis (11) applizierbar ist.

16. Vorrichtung nach Anspruch 15, **dadurch gekennzeichnet, dass** die Strömungsverbindung (13) als flexibler PVC-Schlauch vorliegt.

17. Vorrichtung nach Anspruch 15 oder 16, **dadurch gekennzeichnet, dass** der Entnahmeanschluss (7) zur Strömungsverbindung (13) fluchtet.

18. Vorrichtung nach einem der Ansprüche 15 bis 17, **dadurch gekennzeichnet, dass** der Abtrennbehälter (4) innerhalb eines Mantelgerüsts (14) angeordnet ist und dass das Mantelgerüst (14) so beschaffen ist, dass an der Strömungsverbindung (13) eine Schlauchklemme (18) befestigbar ist.

19. Vorrichtung nach einem der Ansprüche 15 bis 18, **dadurch gekennzeichnet, dass** der Abtrennbehälter (4) zwei zum Entnahmeanschluss (7) beabstandete Zuführanschlüsse (5, 6) für Blut (19) einerseits und für Hydroxyethylstärkelösung (HES) (20) andererseits aufweist.

20. Vorrichtung nach einem der Ansprüche 15 bis 19, **dadurch gekennzeichnet, dass** die Zuführeinrichtung (8, 9) eine innerhalb einer Umhüllung (21) sitzende Spritze (22) mit einem Tubus (23) und mit einem Kolben (24) umfasst und dass der Kolben (24) eine Durchtrittsöffnung (25) aufweist.

21. Vorrichtung nach Anspruch 20, **dadurch gekennzeichnet, dass** von der Umhüllung (21) steril konnektierbare Anschlüsse (27, 32) abragen, die über Absperreinrichtungen (28, 29) öffen- und verschließbar sind.

22. Vorrichtung nach einem der Ansprüche 15 bis 21, **dadurch gekennzeichnet, dass** der Abtrennbehälter (4) einen weiteren steril konnektierbaren Zuführanschluss (43) aufweist, an den eine weitere gegen die Atmosphäre abgedichtete Zuführeinrichtung (44) für einen Füllstoff, insbesondere inerte Kügelchen (45), zur Volumenverminderung in Behälter (12) angeschlossen ist.

## Claims

1. A method for separating blood using a centrifuge, wherein different blood fractions are obtained, wherein a separation container that is to be introduced into the centrifuge is filled with blood through its sterile connectable inlet connection, wherein the blood fraction of interest is removed from the separation container through its sterile connectable withdrawal connection, and wherein a feed device and a withdrawal device each form a closed system during the sterile connection to the separation container,
**characterized in that**
the blood fraction of white blood cells is collected during centrifugation essentially inside a flow connection between two containers inside the separation container and goes from there by means of an interior line disposed in the interior of the withdrawal device.

2. The method according to claim 1, **characterized in that** two feed devices are connected to two feed connections for the feed.

3. The method according to claim 2, **characterized in that** one feed device for blood and one feed device for hydroxyethyl starch solution (HES) are each connected to a feed connection by welding under sterile conditions.

4. The method according to claim 3, **characterized in that** during the filling of the separation container with blood, sterile air goes out of the separation container and into the feed device for HES, or during the filling of the separation container with HES, sterile air goes out of the separation container and into the feed device for blood.

5. The method according to claim 4, **characterized in that** during the blood feed, a vacuum is created by means of the feed device for HES, or during the HES feed, a vacuum is created by means of the feed device for blood.

6. The method according to claim 4 or 5, **characterized in that** the displaced sterile air enters a storage container of the feed device for HES, or blood or is discharged into a pressure equalization device.

7. The method according to claim 6, **characterized in that** the sterile air enters the storage container of the feed device for HES or blood in such a way that it flows past HES or blood.

8. The method according to claim 7, **characterized in that** the area of the flexible storage container of the feed device for HES or blood containing the HES or blood is brought to a distance away from the inlet point into the storage container during the sterile air feed.

9. The method according to any one of claims 1 to 8, **characterized in that** the feed device is disconnected under sterile conditions after successful filling of the separation container.

10. The method according to any one of claims 1 to 9,
**characterized in that** the flow connection, which is in the form of a plastic tube, is separated by means of hose clamps at the point where the phase boundary between the blood fraction of the red blood cells and the blood fraction of the white blood cells is located.

11. The method according to claim 1 to 10, **characterized in that** in addition to the withdrawal of the blood fraction of the white blood cells, plasma is also withdrawn, and the withdrawal device is connected to the withdrawal connection under sterile conditions.

12. The method according to claim 1 to 11, **characterized in that** the internal line is introduced into the flow connection by means of the connections of the withdrawal device and of the separation device that are connected for withdrawal of the blood fraction of white blood cells under sterile conditions.

13. The method according to claim 1 to 12, **characterized in that** the blood fraction of white blood cells, optionally additional plasma, is drawn through a syringe disposed inside the withdrawal device and by means of its piston stroke and into the withdrawal device, which is sealed from the atmosphere, in particular being transferred to a container disposed inside the withdrawal device.

14. The method according to any one of claims 1 to 13, **characterized in that** after successful withdrawal of the blood fraction of white blood cells, plasma from the separation container is optionally disconnected from the withdrawal connection under sterile conditions.

15. A device for separating blood using a centrifuge (1) with a rotor (2), in particular for carrying out the method according to any one of claims 1 to 14, with at least one separation container (4), wherein the separation container (4) has at least one feed connection (5, 6) that can be connected under sterile conditions and has at least one withdrawal connection (7) that can be connected under sterile conditions, to which feed devices and withdrawal devices (8, 9, 10, 44) that are sealed with respect to the atmosphere are connected, wherein the feed device (8, 9, 44) and the withdrawal device (10) are each closed systems and also form a closed system with them during the sterile connection to the corresponding connections (5, 6, 43, 7) of the separation container,
**characterized in that**
the separation container (4) comprises two containers (11, 12) which are flow-connected to one another by means of a flow connection (13), and the withdrawal device (10) has an internal line (36), which can be applied to the flow connection (13) and to the container (11).

16. The device according to claim 15, **characterized in that** the flow connection (13) is in the form of a flexible PVC tube.

17. The device according to claim 15 or 16, **characterized in that** the withdrawal connection (7) is aligned with the flow connection (13).

18. The device according to any one of claims 15 to 17, **characterized in that** the separation container (4) is disposed inside a jacketed structure (14), and the jacketed structure (14) is such that a hose clamp (18) can be attached to the flow connection (13).

19. The device according to any one of claims 15 to 18,
**characterized in that** the separation container (4) has two feed connections (5, 6) for blood (19), on the one hand, and for hydroxyethyl starch solution (HES) (20), on the other hand, these feed connections being spaced a distance apart from the withdrawal connection (7).

20. The device according to any one of claims 15 to 19,
**characterized in that** the feed connection (8, 9) comprises a syringe (22), which sits inside a sheathing (21) and has a tube (23) and a plunger (24), and the plunger (24) has a through-opening (25).

21. The device according to claim 20, **characterized in that** connections (27, 32) that can be connected under sterile conditions and can be opened and closed by means of cutoff devices (28, 29) extend away from the sheathing (21).

22. The device according to any one of claims 15 to 21,
**characterized in that** the separation container (4) has another feed connection (43) which can be connected under sterile conditions and to which another feed connection (44) for a filler, in particular inert beads (45), is connected for reducing the volume in the container (12) and can be sealed with respect to the atmosphere.

## Revendications

1. Procédé de fractionnement de sang en utilisant une centrifugeuse, dans lequel différentes fractions de sang sont obtenues, le remplissage d'un récipient de séparation s'intégrant dans la centrifugeuse avec du sang a lieu par son raccord d'alimentation connectable de manière stérile, le prélèvement de la fraction de sang à laquelle on s'intéresse dans le récipient de séparation ayant lieu par son raccord de prélèvement connectable de manière stérile et un dispositif d'alimentation et un dispositif de prélèvement constituant respectivement un système fermé pendant la connexion stérile avec le récipient de séparation,
**caractérisé en ce que**
la fraction de sang des globules rouges s'accumule pendant la centrifugation sensiblement dans le cadre d'une connexion d'écoulement entre deux récipients à l'intérieur du récipient séparateur et est prélevée de là au moyen d'une conduite interne disposée à l'intérieur du dispositif de prélèvement.

2. Procédé selon la revendication 1, **caractérisé en ce que**, pour l'alimentation, deux dispositifs d'alimentation sont raccordés à deux raccords d'alimentation.

3. Procédé selon la revendication 2, **caractérisé en ce qu'**un dispositif d'alimentation pour du sang et un dispositif d'alimentation pour une solution d'hydroxyéthylamidon (HES) sont soudés de manière stérile respectivement à un raccord d'alimentation.

4. Procédé selon la revendication 3, **caractérisé en ce que**, pendant le remplissage du récipient séparateur avec du sang, de l'air stérile provenant du récipient séparateur arrive dans le dispositif d'alimentation en HES ou que, pendant le remplissage du récipient séparateur avec du HES, de l'air stérile provenant du récipient séparateur arrive dans le dispositif d'alimentation en sang.

5. Procédé selon la revendication 4, **caractérisé en ce que**, pendant l'alimentation en sang, une dépression est créée au moyen du dispositif d'alimentation en HES ou que, pendant l'alimentation en HES, une dépression est créée au moyen du dispositif d'alimentation en sang.

6. Procédé selon la revendication 4 ou 5, **caractérisé en ce que** l'air stérile refoulé arrive dans un réservoir du dispositif d'alimentation en HES ou en sang ou dans un dispositif de compensation de pression.

7. Procédé selon la revendication 6, **caractérisé en ce que** l'air stérile arrive dans le réservoir du dispositif d'alimentation en HES ou en sang de manière à passer devant du HES ou du sang.

8. Procédé selon la revendication 7, **caractérisé en ce que** la partie contenant du HES ou du sang du réservoir souple du dispositif d'alimentation en HES ou en sang est écartée, pendant l'alimentation en air stérile, du point d'entrée dans le réservoir.

9. Procédé selon une des revendications 1 à 8,
**caractérisé en ce que** le dispositif d'alimentation est déconnecté de manière stérile une fois que le récipient séparateur est rempli.

10. Procédé selon une des revendications 1 à 9, **caractérisé en ce que** le connecteur d'écoulement se présentant sous forme d'un tuyau plastique est serré au moyen d'un collier de serrage à l'endroit où se trouve la limite de phase entre la fraction sanguine des globules rouges et la fraction sanguine des globules blancs.

11. Procédé selon une des revendications 1 à 10,
**caractérisé en ce que**, en plus du prélèvement de la fraction sanguine des globules blancs, du plasma est aussi prélevé et que le dispositif de prélèvement est connecté de manière stérile au raccord de prélèvement.

12. Procédé selon une des revendications 1 à 11,
**caractérisé en ce que** la conduite interne est intégrée par l'intermédiaire des raccords à connexion stérile pour le prélèvement de la fraction sanguine et les globules blancs du dispositif de prélèvement et du récipient séparateur dans le connecteur d'écoulement.

13. Procédé selon une des revendications 1 à 12,
**caractérisé en ce que** la fraction sanguine des globules blancs, éventuellement du plasma complémentaire, est aspirée par une seringue disposée à l'intérieur du dispositif de prélèvement et la course de son piston et transférée dans le dispositif de prélèvement isolé de l'atmosphère, en particulier dans un récipient disposé à l'intérieur du dispositif de prélèvement.

14. Procédé selon une des revendications 1 à 13,
**caractérisé en ce que** le dispositif de prélèvement, après le prélèvement de la fraction sanguine des globules blancs, éventuellement du plasma, dans le récipient séparateur est déconnecté de manière stérile du raccord cinq minutes de de prélèvement.

15. Procédé de fractionnement de sang en utilisant une centrifugeuse (1) dotée d'un rotor (2), en particulier pour la réalisation du procédé selon une des revendications 1 à 14, d'au moins un récipient séparateur (4), dans lequel le récipient séparateur (4) présente au moins un raccord d'alimentation (5, 6) connectable de manière stérile et au moins un raccord de prélèvement (7) connectable de manière stérile auxquels des dispositifs d'alimentation et de prélèvement (8, 9, 10, 44) isolés de l'atmosphère sont raccordés de manière stérile, le dispositif d'alimentation (8, 9, 44) et le dispositif de prélèvement (10) sont des systèmes fermés et constituent également pendant la connexion stérile, avec les raccords correspondants (5, 6, 43, 7) du récipient séparateur, en commun avec celui-ci, un système fermé,
**caractérisé en ce que**
le récipient séparateur (4) comprend deux réceptacles (11, 12) qui sont reliés entre eux au niveau écoulement par un connecteur d'écoulement (13) et que le dispositif de prélèvement (10) présente une conduite interne (36) qui est applicable dans le connecteur d'écoulement (13) et dans le réceptacle (11).

16. Dispositif selon la revendication 15, **caractérisé en ce que** le connecteur d'écoulement (13) se présente sous forme d'un tuyau en PVC souple

17. Dispositif selon la revendication 15 ou 16,
**caractérisé en ce que** le raccord de prélèvement (7) est aligné par rapport au connecteur d'écoulement (13).

18. Dispositif selon une des questions 15 à 17,
**caractérisé en ce que** le récipient séparateur (4) est disposé à l'intérieur d'une structure d'enveloppe (14) et que la structure d'enveloppe (14) est réalisée de manière à ce qu'un collier de serrage (18) puisse être fixé au connecteur d'écoulement (13).

19. Dispositif selon une des questions 15 à 18,
**caractérisé en ce que** le récipient séparateur (4) présente deux raccords d'alimentation (5, 6) pour du sang (19) espacés du raccord de prélèvement (7) d'une part et pour de la solution hydroxyéthylamidon (HES) (20) d'autre part.

20. Dispositif selon une des questions 15 à 19,
**caractérisé en ce que** le dispositif d'alimentation (8, 9) présente une seringue (22) posée dans une enveloppe (21) et comportant un tube (23) et un piston (24) et que le piston (24) présente un orifice de passage (25).

21. Dispositif selon la revendication 20, **caractérisé en ce que** dépassent de l'enveloppe (21) des raccords (27, 32) connectables de manière stérile qui peuvent être ouverts et fermés au moyen de dispositifs de blocage (28, 29).

22. Dispositif selon une des questions 15 à 21,
**caractérisé en ce que** le récipient séparateur (4) présente un autre raccord d'alimentation (43) connectable de manière stérile auquel se raccorde un autre dispositif d'alimentation (44) isolé de l'atmosphère pour une matière de charge, en particulier des billes inertes (45) pour réduire le volume dans le récipient (12).
